Europäisches Patentamt

European Patent Office

Office européen des brevets

(11) Publication number: **0 121 571**
**B1**

# EUROPEAN PATENT SPECIFICATION

(45) Date of publication of patent specification: **04.04.90**

(21) Application number: **83903207.5**

(22) Date of filing: **12.10.83**

(86) International application number:
**PCT/JP83/00340**

(87) International publication number:
**WO 84/01514 26.04.84 Gazette 84/11**

(51) Int. Cl.⁵: **A 61 M 25/00, A 61 B 17/42**

(54) **INDWELLING CATHETER FOR CERVICAL CANAL.**

(30) Priority: **12.10.82 JP 177599/82**

(43) Date of publication of application:
**17.10.84 Bulletin 84/42**

(45) Publication of the grant of the patent:
**04.04.90 Bulletin 90/14**

(84) Designated Contracting States:
**DE FR GB SE**

(56) References cited:
**DE-A-2 454 589**
**FR-A-2 380 033**
**JP-A-5 431 985**
**US-A-3 543 758**
**US-A-4 100 923**
**US-A-4 198 981**
**US-A-4 351 342**

(73) Proprietor: **SUMITOMO BAKELITE COMPANY LIMITED**
**2-2, Uchisaiwaicho 1-chome Chiyoda-ku Tokyo 100 (JP)**

(73) Proprietor: **OGITA, Sachio**
**9-19 Amamihigashi 6-chome**
**Matsubara-shi Osaka 580 (JP)**

(72) Inventor: **OGITA, Sachio**
**9-19, Amamihigashi 6-chome**
**Matsubara-shi Osaka 580 (JP)**

(74) Representative: **Carter, Caroline Ann et al**
**ABEL & IMRAY Northumberland House 303-306 High Holborn**
**London WC1V 7LH (GB)**

Courier Press, Leamington Spa, England.

# Description

This invention relates to a cervical canal catheter designed in order not only to prevent possible complications accompanying the premature rupture of the membrane before term in a pregnant woman, but also to carry out extraction after awaiting the maturity of a fetus.

The premature rupture of the membrane before the beginning of parturition causes an efflux of amniotic fluid from the ruptured part, an onset of labour-pains accompanying the amniorrhea and an ascending intrauterine infection from the ruptured part, and before normal gestation (40 weeks), it follows that an immature fetus is born. Therefore, both the mother and the fetus are exposed to serious danger.

Treatment of this condition is roughly divided into two methods, positive induction of labour and watchful waiting. The former is based on the assertion that prompt delivery of a fetus followed by care in an incubator is better than exposure to a danger of microbism that may be caused sooner or later, and the latter is a method by which extraction is carried out after the maturation of a fetus has been prolonged as long as possible by intravaginal disinfection and administration of antibiotics and tocolytic agents to the mother's body.

However, in the case of the former, the function of the lungs (respiratory function) of the fetus is always immature before 35 weeks of gestation, and therefore, there is a risk of respiratory distress syndrome immediately after the birth, so that the life of the fetus is endangered. In the case of the latter, it is found that one effect of the stress imposed on the fetus by rupture of the membrane is that maturation of the function of the lungs of the fetus is accelerated, but the efflux of amniotic fluid cannot but be looked on, and there is always a danger of infection in the amniotic fluid. Recently, in order to prevent infection in amniotic fluid, reports have successively been made on attempts to prevent ascending infection from vagina to uterus by putting a contraceptive pessary on cervix uteri and always injecting thereinto an antiseptic solution. This method permits the prevention to some extent of the ascending infection after the attachment of the pessary, but is ineffective when infection in the amniotic fluid has already occurred (usually, bacteria are detected in the amniotic fluid 2 hours after rupture of membrane) and is ineffective for preventing the amniotic fluid from effusing because no close adhesion can be attained between the cervix uteri and the pessary.

An intrauterine environment is more perfect and comfortable an incubator than any artificial incubator for a fetus; the fetus in the mother's body is supplied with nutrients and oxygen by the mother body through the placenta as a point of contact. If complications accompanying the rupture of the membrane can be prevented, there will be brought about an epoch-making reform in the control of mother and fetus. The present inventors have found that premature rupture of membrane for which no suitable remedy has heretofore been available can be kept under positive control by attaching a cervical canal catheter so designed in order to satisfy the above-mentioned clinical requirements, by hysterotrachelorrhaphy (surgical closure of the cervix) which is used for cervical incompetency.

U.S. Patent Specification No. 4 351 342 discloses a urinary catheter which makes possible drainage of urine into one or more drainage holes without drawing body tissue into the catheter tube and in which the drainage holes and the distal end of the tube are cushioned so as to prevent internal inflammation of the patient. The catheter comprises an elongate tube with a closed distal end and a urine-drainage hole in the side wall of the tube adjacent to the closed end. A balloon envelopes the closed end and is so shaped as to provide an opening which is a tapered lead-in for the drainage hole. The balloon also cushions the tip of the catheter, thus allowing the tip to be relatively stiff to facilitate insertion of the catheter through the urethra by introducing a metal rod into the tube to bear on the closed distal end.

The invention provides a cervical canal catheter comprising a catheter main tube made of a soft rubber or resin and having an open distal end, a gourd-shaped balloon having a narrow centre part or two balloons axially spaced from each other, the balloon(s) being positioned around the main tube at or in contiguity to the said open end, each balloon communicating via a balloon injection-hole with a balloon injection-passage in the wall of the main tube, or formed integrally with the main tube, for injecting a gas or liquid under pressure into the balloon, and there being a liquid-medicine-injecting passage in the wall of the main tube or formed integrally with the main tube, which liquid-medicine-injecting passage communicates with the exterior of the catheter in the narrow part of the gourd-shaped balloon or between the two balloons, the balloon injecting-passage(s) and the liquid-medicine-injecting passage branching off from the main tube at the end of the main tube remote from the said open end.

The open end of the catheter main tube is advantageously provided with a trumpet-shaped film in contiguity to the gourd-shaped balloon or, if two balloons are present, to the balloon nearer to the open end.

Preferably, the gourd-shaped balloon, or the balloon nearer to the open end, juts out beyond the open end of the catheter main tube when expanded.

In a preferred embodiment of the invention, the or each balloon injection passage branches off from the main tube as a balloon injection tube and the or each said branch tube is provided with a check valve and a pilot balloon, the liquid-medicine-injecting passage branching off from the main tube in the form of a liquid-medicine injecting branch tube which is provided with a cap, the end of the catheter main tube remote from the open end also being provided with a cap. Where the catheter comprises two balloons, each preferably communicates with a separate balloon injection passage.

Advantageously, a sampling hole is provided in the wall of the main tube to provide communication between the exterior of the catheter and the main cavity of the main tube.

In a catheter of the invention having two balloons, the liquid-medicine-injecting passage in the wall of the main tube advantageously communicates with the exterior of the catheter via a liquid-medicine-injecting hole provided in the catheter main tube, the liquid-medicine-injecting hole being located between the two balloons. Where there is a gourd-shaped balloon, the liquid-medicine-injecting passage preferably extends from the main tube as a tube which terminates as a liquid-medicine-injecting hole in the vicinity of the narrow part of the gourd-shaped balloon.

Advantageously, the main cavity of the main tube is substantially unobstructed.

Two embodiments of the present invention will now be described, by way of example only, with reference to the accompanying drawings, in which:

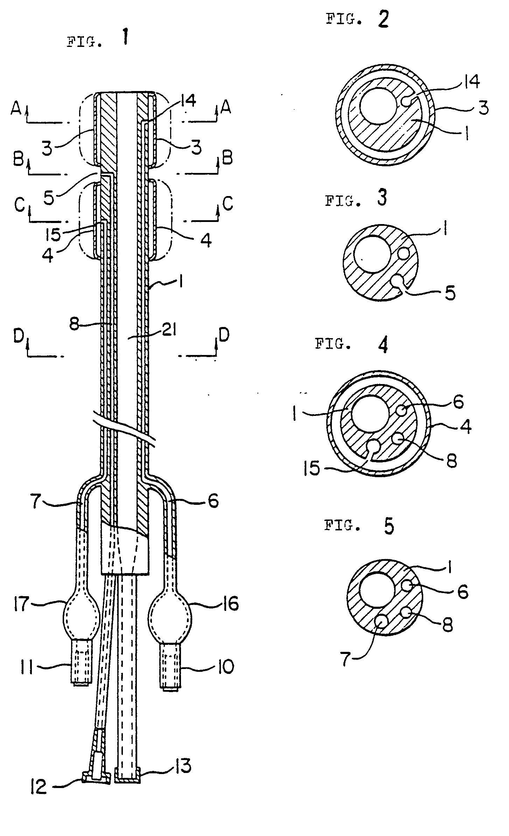

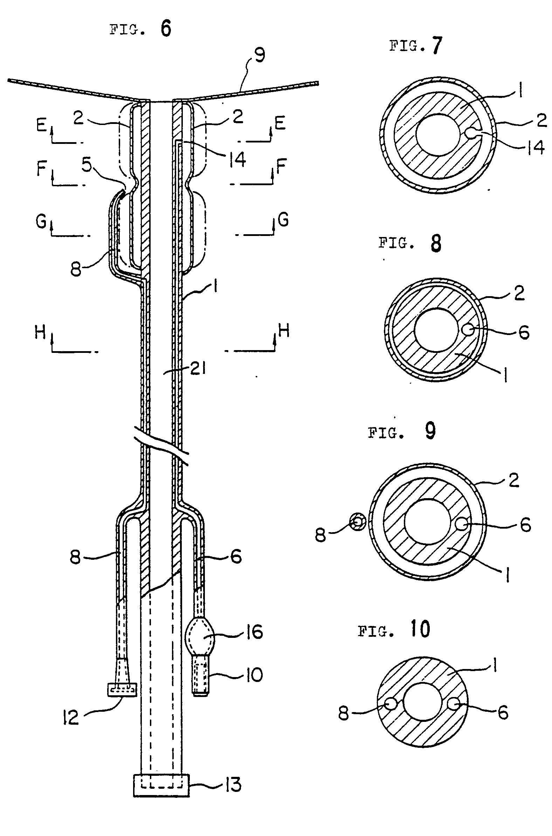

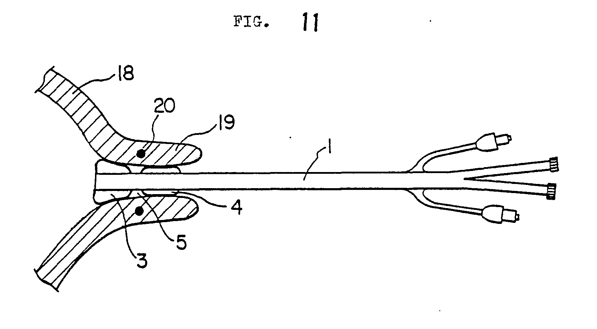

Fig. 1 is a longitudinal section through a first embodiment of this invention and Figs. 2 to 5 are cross-sectional views along the A-A line, the B-B line, the C-C line and the D-D line, respectively, in Fig. 1 indicating the actual arrangement of the passages. Fig. 6 is a longitudinal section through a second embodiment of this invention, Figs. 7 to 10 are cross-sectional views along the E-E line, the F-F line, the G-G line and the H-H line, respectively, in Fig. 6, and Fig. 11 is an explanatory view showing the use of the cervical canal catheter according to this invention. In the drawings, 1 shows a catheter main tube, 2 a gourd-shaped balloon, 3 and 4 balloons, 5 a liquid-medicine-injecting hole, 6 and 7 balloon injection-passages, 8 a liquid-medicine-injecting passage, 9 a trumpet-shaped film, 10 and 11 check valves, 12 and 13 caps, 14 and 15 balloon injection-holes, 16 and 17 pilot balloons, 18 a uterus, 19 a cervical canal, 20 a ligature thread, and 21 the main cavity of the catheter main tube.

Fig. 1 and Fig. 6 are longitudinal sections through two embodiments of this invention. A gourd-shaped balloon 2 (Fig. 6) having a narrow part in the middle or two balloons 3 and 4 (Fig. 1) which are independent of each other in the axial direction are provided around a catheter main tube 1 made of a soft rubber or resin having an open end, the gourd-shaped balloon or two balloons being positioned at the tip of or in contiguity to the tip of the catheter main tube. A liquid-medicine-injecting hole 5 is provided in the narrow part of the gourd-shaped balloon 2 or in the valley between the two independent balloons 3 and 4. Balloon injection-holes 14 and 15 and balloon injection-passages 6 and 7 for injecting a gas or liquid under pressure into the balloons and a liquid-medicine-injecting passage 8 leading to the liquid-medicine-injecting hole 5 are provided in the wall of the catheter main tube 1 or integrally with the catheter main tube 1. At the other end of the catheter main tube, the balloon injection-passages 6 and 7 and the liquid-medicine-injecting passage 8 branch off from the catheter main tube 1. The cervical canal catheter of the invention preferably also comprises a trumpet-shaped film at the open end portion of the catheter main tube 1 in contiguity to the balloon.

As the soft rubber or resin there may be used any of those materials used generally in medical instruments, for example, soft vinyl chloride resin, natural rubber, silicone rubber, urethane rubber or the like; silicone rubber is more suitable than the others because the catheter is allowed to indwell for a long time and the catheter main tube is preferably transparent. The balloon or balloons attached around the catheter main tube 1 in contiguity to the tip of the catheter main tube 1 may be made of a material which is either the same as or different from the catheter main tube in quality. The balloon may be gourd-shaped having a narrow part in the middle as shown in Fig. 6 or may be separated into two balloons 3 and 4 as shown in Fig. 1. What is important is that a narrow valley is formed at the centre portion of the two swellings of the balloon or balloons when the balloon or balloons are expanded, and the liquid-medicine-injecting hole 5 is provided in the valley. Moreover, it is desirable that the balloon juts out beyond the tip of the catheter main tube 1 upon expansion. The balloon is attached in contiguity to the tip of the catheter main tube 1, and the length of the whole balloon is preferably 50 mm or less. Each balloon has a balloon injection-hole 14 or 15 in the catheter main tube 1 inside the balloon, and the balloon injection-passages 6 and 7 branch off from the catheter main tube 1 at the other end. To the ends of the injection passages 6 and 7 are preferably attached pilot balloons 16 and 17, respectively, and check valves 10 and 11, respectively. The balloons can be expanded by injecting a gas or liquid under pressure through the respective check valves by means of a syringe, and the turgor condition can be determined by means of a pilot balloon. Since the gourd-shaped balloon 2 shown in Fig. 6 is expanded through the single balloon injection-passage 6, the inside pressure of the whole balloon is uniform and the balloon is expanded in the form of a gourd. When two separate balloons 3 and 4 are used, the respective balloon injection-passages 6 and 7 are provided independently, with the advantage that the degree of expansion of the two balloons can be adjusted individually.

The liquid-medicine-injecting hole 5 provided in the valley between the swellings of the balloon or balloons is formed at the end of a liquid-medicine-injecting passage 8 in the wall of the catheter main tube 1 in the embodiment of Fig. 1, but may be formed by leading a fine tube along the outer wall of the balloon as in the case of the gourd-shaped balloon 2 shown in the embodiment in Fig. 6. The other end of the liquid-medicine-injecting passage 8 branches off from the catheter main tube 1, and a cap 12 is attached to the end. It is preferable to attach a cap 13 to the other end of the catheter main tube 1 in order to prevent any infection during the indwelling. The catheter main tube 1 may be provided with a sampling hole at any position along the main cavity 21 for collecting a humor discharged from the open end.

It is preferred that the main cavity 21 of the catheter main tube 1 has such a structure that a fine tube for sampling an amniotic fluid sample can, if necessary, be inserted up to open end.

In Fig. 11, an explanatory view is given for showing the method of use of the cervical canal-indwelling catheter according to this invention. The balloons 3 and 4, by means of their turgor, provide better adherence of the catheter to the inner surface of a cervical canal, when the canal is subjected to plication and narrowing by surgical ligature and the catheter is allowed to indwell in such a position that the strangulation ring formed by use of a ligature thread coincides in position with the valley between the two balloons 3 and 4. Therefore, an effect of fixing the catheter in the cervical canal is brought about by the ligaturing force and the turgor of the balloons, so that indwelling of the catheter in the cervical canal which has heretofore been impossible to imagine has become possible.

The trumpet-shaped film 9 optionally provided in the open end portion of the catheter main tube 1 in contiguity to the balloon is preferably a flexible orbicular or conical film having a thickness of about 1 mm or less and a diameter of 100 mm or less, and its diameter can be adjusted by cutting the film depending on the degree of opening of the uterus of a patient. The trumpet-shaped film 9 is used for covering a velamentum which has been physically damaged. The film, because it is placed along the curve of the interior of the uterus, prevents efflux of amniotic fluid from the damaged portions and prevents ascent of mucus and bacteria from the cervix uteri, and uniformly disperses the rise of the intrauterine pressure by Laplace's theorem to prevent the direction of pressure from being concentrated into the cervix uteri.

As described above, the uterus is physically blocked with the balloons 3 and 4 or the balloon 2 to isolate its inside from the outside, so that it becomes possible to prevent not only the efflux of amniotic fluid but also infection by bacteria. Further, an antiseptic solution introduced from the liquid-medicine-injecting hole 5 into the valley between the two balloons 3 and 4 or into the narrow part at the centre portion of the gourd-shaped balloon 2 shown in the embodiment in Fig. 6 fills the space between the valley or narrow part and the inner wall of the cervical canal, and therefore, even if bacteria invade from the vagina side, they will undergo sterilization in this space. The cervical mucous passes through the space filled with the antiseptic solution and is introduced, together with the antiseptic solution overflow, into the vagina while wetting the inner surface of the cervical canal on the vagina side in relation to the balloon 4 or the gourd-shaped balloon 2.

Since the branch at the other end of the transparent catheter main tube 1 protrudes from the vagina, the properties, in particular, the colour of the amniotic fluid filling the catheter can easily be ascertained. Therefore, threatened asphyxia (excretion of meconium) can be diagnosed without using an amnioscope. Further, an amniotic fluid sample indispensable for the management of a fetus (judgement of the maturation degree of the fetus) can easily be collected at any time from the main cavity 21 of the catheter main tube 1 without need for a conventional transabdominal amniocentesis. It is also possible to collect the amniotic fluid sample by taking off the cap 13 of the catheter main tube 1, inserting a fine tube into the main cavity 21 through the opening at the tip until it reaches the inner part of the uterus, and then collecting the sample through said fine tube.

Even in a case in which a long time has elapsed after the rupture of membrane, the attachment of the catheter according to this invention makes it possible to take off the cap 13 at the other end of the catheter main tube 1 and inject an antibiotic therefrom directly into the amniotic cavity. Therefore, the attachment makes it possible to increase the concentration of the antibiotic in the amniotic fluid certainly as compared with conventional administration through the mother body, and can exhibit effects in the case where infection has already occurred (this can be diagnosed from the number of leukocytes in the amniotic fluid and by microscopic examination of bacteria). Further, the attachment enables direct administration to the fetus of pulmonary maturation promotors (steroid hormones, etc.) or drugs necessary for the management of a fetus and it is expected to establish a way to conduct prenatal treatment which is now the chief desire of perinatal medicine.

As described above, when used together with hysterotrachelorrhaphy, the cervical canal catheter for premature rupture of membrane according to this invention can be expected to bring about the following effects:

1. In controlling the premature rupture of membrane, efflux of amniotic fluid and concomitant onset of labour pains can be prevented.

2. The infection in amniotic fluid, previously unavoidable in this disease, can be prevented.

3. The properties of amniotic fluid can visually be observed, and an amniotic fluid sample necessary for the analysis of the amniotic fluid can easily be collected.

4. Threatened asphyxia or fetal infection can be diagnosed easily and accurately.

5. Even when infection has already occurred, the chance of subsequent infection is removed, and moreover very effective treatment is made possible by administration of an antibiotic into the amniotic cavity.

6. Direct administration of fetal pulmonary maturation promotors becomes possible, so that therapeutic effects can be improved.

7. By co-use of a tocolytic agent, a fetus can be safely managed until it acquires an ability to adapt to the postnatal environment.

8. Since amniotic fluid can be analyzed frequently, the physiology of fetal growth which has not yet been elucidated can be ascertained.

9. Attachment of the catheter of the invention makes it possible greatly to reduce the need for rest of patients with said condition who have heretofore required absolute rest owing to the necessity for the Trendelenburg position, and the patients can be allowed to walk or even attend a hospital as outpatients depending on the particular case.

The invention also provides a kit comprising a catheter in accordance with the invention and a tube which can be inserted into the main cavity of the catheter main tube at the end remote from the open end and can extend to the open end.

**Claims**

1. A cervical canal catheter comprising a catheter main tube (1) made of a soft rubber or resin and having an open distal end, a gourd-shaped balloon (2) having a narrow centre part or two balloons (3, 4) axially spaced from each other, the balloon(s) being positioned around the main tube at or in contiguity to the said open end, each balloon communicating via a balloon injection-hole (14, 15) with a balloon injection-passage (6, 7) in the wall of the main tube, or formed integrally with the main tube, for injecting a gas or liquid under pressure into the balloon, and there being a liquid-medicine-injecting passage (8) in the wall of the main tube or formed integrally with the main tube, which liquid-medicine-injecting passage communicates with the exterior of the catheter in the narrow part of the gourd-shaped balloon or between the two balloons, the balloon injecting-passage(s) and the liquid-medicine-injecting passage branching off from the main tube at the end of the main tube remote from the said open end.

2. A cervical canal catheter as claimed in claim 1, wherein the open end of the catheter main tube is provided with a trumpet-shaped film (9) in contiguity to the gourd-shaped balloon or, if two balloons are present, to the balloon nearer to the open end.

3. A cervical canal catheter as claimed in claim 1 or 2, wherein the gourd-shaped balloon, or the balloon nearer to the open end, juts out beyond the open end of the catheter main tube when expanded.

4. A cervical canal catheter as claimed in any one of claims 1 to 3, wherein the or each balloon injection passage branches off from the main tube as a balloon injection branch tube and the or each said branch tube is provided with a check valve (10, 11) and a pilot balloon (16, 17), wherein the liquid-medicine-injecting passage (8)

branches off from the main tube in the form of a liquid-medicine-injecting branch tube and the said liquid-medicine-injecting branch tube is provided with a cap (12), and wherein the end of the catheter main tube remote from the open end is provided with a cap (13).

5. A cervical canal catheter as claimed in any one of claims 1 to 4, wherein there are two balloons each of which communicates with a separate balloon injection passage.

6. A cervical canal catheter as claimed in any one of claims 1 to 5, wherein a sampling hole is provided in the wall of the main tube to provide communication between the exterior of the catheter and the main cavity of the main tube.

7. A cervical canal catheter as claimed in any one of claims 1 to 6 and having two balloons, wherein the liquid-medicine-injecting passage in the wall of the main tube communicates with the exterior of the catheter via a liquid-medicine-injecting hole (5) provided in the catheter main tube, the liquid-medicine-injecting hole being located between the two balloons.

8. A cervical canal catheter as claimed in any one of claims 1 to 6 and having a gourd-shaped balloon, wherein the liquid-medicine injecting passage extends from the main tube as a tube which terminates as a liquid-medicine-injecting hole (5) in the vicinity of the narrow part of the gourd-shaped balloon.

9. A cervical canal catheter as claimed in any one of claims 1 to 8, wherein the main cavity (21) of the main tube is substantially unobstructed.

10. A kit comprising a cervical canal catheter as claimed in any one of claims 1 to 9 and a tube which can be inserted into the main cavity of the catheter main tube at the end remote from the open end and can extend to the open end.

**Patentansprüche**

1. Katheter für den Cervix-Kanal, umfassend einen Katheter-Hauptschlauch (1) aus einem weichen Gummi bzw. Kautschuk oder Kunstharz mit einem offenen distalen Ende, einen flaschenkürbisförmigen Ballon (2) mit einem verengten bzw. eingeschnürten Mittelteil oder zwei axial auf Abstand voneinander angeordneten Ballons (3, 4), der bzw. die um den Hauptschlauch herum am offenen Ende oder im Anschluß an dieses angeordnet ist bzw. sind, wobei jeder Ballon über eine Balloneinleitöffnung (14, 15) mit einem in der Wand des Hauptschlauches vorgesehenen oder materialeinheitlich mit dem Hauptschlauch ausgebildeten Balloneinleitdurchgang (6, 7) zum Einleiten eines Gases oder einer Flüssigkeit unter Druck in den Ballon kommuniziert, wobei ein Flüssigmedizin-Injizierdurchgang (8) in der Wand des Hauptschlauches vorgesehen oder materialeinheitlich mit dem Hauptschlauch ausgebildet ist, welcher Flüssigmedizin-Injizierdurchgang mit der Außenseite des Katheters im eingeschnürten Teil des flaschenkürbisförmigen Ballons oder zwischen den beiden Ballons in Verbindung steht, und wobei der (die) Balloneinleitdurchgang bzw.

-durchgänge und der Flüssigmedizin-Injizierdurchgang an dem vom offenen Ende entfernten Ende des Hauptschlauches von diesem abzweigen.

2. Katheter für den Cervix-Kanal nach Anspruch 1, dadurch gekennzeichnet, daß das offene Ende des Katheter-Hauptschlauches mit einem trompetenförmigen Film (9) versehen ist, der sich unmittelbar an den flaschenkürbisförmigen Ballon oder im Fall von zwei Ballons an den näher am offenen Ende gelegenen Ballon anschließt.

3. Katheter für den Cervix-Kanal nach Anspruch 1 oder 2, dadurch gekennzeichnet, daß der flaschenkürbisförmige Ballon oder der dem offenen Ende nähergelegene Ballon im aufgeweiteten Zustand über das offene Ende des Katheter-Hauptschlauches hinaus vorsteht.

4. Katheter für den Cervix-Kanal nach einem der Ansprüche 1 bis 3, dadurch gekennzeichnet, daß der oder jeder Balloneinleitdurchgang vom Hauptschlauch als ein Balloneinleitzweigschlauch abzweigt und der oder jeder Zweigschlauch mit einem Rückschlagventil (10, 11) und einem Steuerballon (16, 17) versehen ist, daß der Flüssigmedizin-Injizierdurchgang (8) vom Hauptschlauch in Form eines Flüssigmedizin-Injizierzweigschlauches abzweigt, der mit einer (Verschluß-)Kappe (12) versehen ist, und daß das vom offenen Ende entfernte Ende des Katheter-Hauptschlauches mit einer (Verschluß-)Kappe (13) versehen ist.

5. Katheter für den Cervix-Kanal nach einem der Ansprüche 1 bis 4, dadurch gekennzeichnet, daß zwei Ballons vorgesehen sind, von denen jeder mit einem getrennten Balloneinleitdurchgang kommuniziert.

6. Katheter für den Cervix-Kanal nach einem der Ansprüche 1 bis 5, dadurch gekennzeichnet, daß in der Wand des Hauptschlauches eine Probenöffnung zur Herstellung einer Verbindung zwischen der Außenseite des Katheters und dem Haupthohlraum oder -kanal des Hauptschlauches vorgesehen ist.

7. Katheter für den Cervix-Kanal nach einem der Ansprüche 1 bis 6, mit zwei Ballons, dadurch gekennzeichnet, daß der Flüssigmedizin-Injizierdurchgang in der Wand des Hauptschlauches mit der Außenseite des Katheters über eine im Katheter-Hauptschlauch ausgebildete Flüssigmedizin-Injizieröffnung (5), die zwischen den beiden Ballons angeordnet ist, kommuniziert.

8. Katheter für den Cervix-Kanal nach einem der Ansprüche 1 bis 6, mit einem flaschenkürbisförmigen Ballon, dadurch gekennzeichnet, daß der Flüssigmedizin-Injizierdurchgang vom Hauptschlauch als ein Schlauch abgeht, der als Flüssigmedizin-Injizieröffnung (5) in der Nähe des eingeschnürten Teils des flaschenkürbisförmigen Ballons endet.

9. Katheter für den Cervix-Kanal nach einem der Ansprüche 1 bis 8, dadurch gekennzeichnet, daß der Haupthohlraum oder -kanal (21) des Hauptschlauches praktisch unversperrt bzw. durchgängig ist.

10. (Bausatz-)Ausrüstung mit einem Cervixkanal-Katheter nach einem der Ansprüche 1 bis 9 und einem Schlauch, der in den Haupthohlraum oder -kanal des Katheter-Hauptschlauches an dem vom offenen Ende entfernten Ende einführbar ist und sich bis zum offenen Ende erstrecken kann.

**Revendications**

1. Cathéter pour canal cervical, caractérisé en ce qu'il comprend un tube cathéter principal (1) formé d'une résine ou d'un caoutchouc mou et ayant une extrémité distale ouverte, un ballon (2) en forme de gourde ayant une partie centrale étroite ou deux ballons (3, 4) distants axialement l'un de l'autre, le ballon ou les ballons étant placés autour du tube principal à l'extrémité ouverte ou sous forme contiguë à l'extrémité ouverte, chaque ballon communiquant, par un trou (14, 15) d'injection dans le ballon, avec un passage (6, 7) d'injection dans le ballon formé dans la paroi du tube principal ou formé en une seule pièce avec le tube principal afin qu'un gaz ou un liquide sous pression puisse être injecté dans le ballon, un passage (8) d'injection d'un liquide médicinal étant formé dans la paroi du tube principal ou en une seule pièce avec le tube principal, le passage d'injection d'un liquide médicinal communiquant avec l'extérieur du cathéter dans la partie étroite du ballon en forme de gourde ou entre les deux ballons, le passage ou les passages d'injection dans les ballons et le passage d'injection d'un liquide médicinal partant du tube principal à l'extrémité du tube principal qui est distante de l'extrémité ouverte.

2. Cathéter pour canal cervical selon la revendication 1, dans lequel l'extrémité ouverte du tube cathéter principal a un film (9) en forme d'entonnoir qui est contigu au ballon en forme de gourde ou, lorsque deux ballons sont présents, au ballon le plus proche de l'extrémité ouverte.

3. Cathéter pour canal cervical selon la revendication 1 ou 2, dans lequel le ballon en forme de gourde ou le ballon le plus proche de l'extrémité ouverte est en saillie au-delà de l'extrémité ouverte du tube cathéter principal lorsqu'il est dilaté.

4. Cathéter pour canal cervical selon l'une quelconque des revendications 1 à 3, dans lequel le passage d'injection dans un ballon ou chaque passage d'injection dans un ballon part en dérivation du tube principal sous forme d'un tube en dérivation d'injection dans un ballon et le tube en dérivation ou chaque tube en dérivation a un clapet de retenue (10, 11) et un ballon pilote (16, 17), le passage d'injection de liquide médicinal (8) partant en dérivation du tube principal sous forme d'un tube en dérivation d'injection de liquide médicinal et ce tube de dérivation d'injection d'un liquide médicinal est muni d'un capuchon (12), et l'extrémité du tube cathéter principal distante de l'extrémité ouverte est munie d'un capuchon (13).

5. Cathéter pour canal cervical selon l'une quelconque des revendications 1 à 4, dans lequel deux ballons sont présents, chacun communiquant avec un passage séparé d'injection dans le ballon.

6. Cathéter pour canal cervical selon l'une quelconque des revendications 1 à 5, dans lequel un trou d'échantillonnage est formé dans la paroi du tube principal afin qu'il assure la communication entre l'extérieur du cathéter et la cavité principale du tube principal.

7. Cathéter pour canal cervical selon l'une quelconque des revendications 1 à 6, comportant deux ballons, dans lequel le passage d'injection d'un liquide médicinal formé dans la paroi du tube principal communique avec l'extérieur du cathéter par un trou (5) d'injection d'un liquide médicinal formé dans le tube cathéter principal, le trou d'injection d'un liquide médicinal étant placé entre les deux ballons.

8. Cathéter pour canal cervical selon l'une quelconque des revendications 1 à 6, comportant un ballon en forme de gourde, dans lequel le passage d'injection d'un liquide médicinal part du tube principal sous forme d'un tube qui aboutit à un trou (5) d'injection d'un liquide médicinal placé au voisinage de la partie étroite du ballon en forme de gourde.

9. Cathéter pour canal cervical selon l'une quelconque des revendications 1 à 8, dans lequel la cavité principale (21) du tube principal ne comporte pratiquement aucun obstacle.

10. Kit comprenant un cathéter pour canal cervical selon l'une quelconque des revendications 1 à 9, et un tube qui peut être introduit dans la cavité principale du tube cathéter principal à l'extrémité distante de l'extrémité ouverte et qui peut rejoindre l'extrémité ouverte.

FIG. 1

FIG. 2

FIG. 3

FIG. 4

FIG. 5

EP 0 121 571 B1

FIG. 6

FIG. 7

FIG. 8

FIG. 9

FIG. 10

FIG. 11